# EUROPÄISCHE PATENTANMELDUNG

(11) **EP 4 197 741 A1**
(43) Veröffentlichungstag der Anmeldung: **21.06.2023**
(21) Anmeldenummer: 22208377.6
(22) Anmeldetag: 18.11.2022
(51) Int. Cl.: B29C 64/106, A61F 2/78, B29C 64/393, B29C 64/40, B33Y 10/00, B33Y 50/02, B33Y 80/00, B29L 31/00

(54) **VERFAHREN ZUM HERSTELLEN EINES 3-DIMENSIONALEN OBJEKTES UND VORRICHTUNG DAZU**

(30) Priorität: 17.12.2021 DE 102021133749
(71) Anmelder: Ottobock SE & Co. KGaA, 37115 Duderstadt (DE)
(72) Erfinder: KROLL-ORYWAHL, Olaf, 37115 Duderstadt (DE); FINKE, Lars Benjamin, 37115 Duderstadt (DE); VIER, Leonard, 37115 Duderstadt (DE)
(74) Vertreter: Gramm, Lins & Partner Patent- und Rechtsanwälte PartGmbB

(57) **Zusammenfassung**

Die Erfindung betrifft ein Verfahren zum Herstellen eines 3-dimensionalen Objektes (6) mittels eines additiven Fertigungsverfahrens, bei dem aus wenigstens einer Einbringöffnung einer Einbringnadel wenigstens ein Fertigungsmaterial in einem fließfähigen Zustand in ein Stützmaterial (4) eingebracht wird und danach aushärtet, wobei das Fertigungsmaterial nach dem Aushärten elastisch ist, wobei die Kontur und/oder die Position wenigstens eines Teils des 3-dimensionalen Objektes innerhalb des Stützmaterials mittels wenigstens eines Sensors (8,14) erfasst wird.

## Beschreibung

Die Erfindung betrifft ein Verfahren zum Herstellen eines 3-dimensionalen Objektes mittels eines additiven Fertigungsverfahrens, bei dem aus wenigstens einer Einbringöffnung einer Einbringnadel wenigstens ein Fertigungsmaterial in einem fließfähigen Zustand in ein Stützmaterial eingebracht wird und danach aushärtet, wobei das Fertigungsmaterial nach dem Aushärten elastisch ist. Die Erfindung betrifft zudem eine Vorrichtung zum Durchführen eines derartigen Verfahrens, wobei die Vorrichtung einen Behälter, der mit Stützmaterial gefüllt oder füllbar ist, und wenigstens eine Einbringnadel aufweist, durch die ein Fertigungsmaterial in den Behälter einbringbar ist.

Additive Fertigungsverfahren sind heute in vielen Formen aus dem Stand der Technik bekannt und werden zur Herstellung sehr unterschiedlicher 3-dimensionaler Objekte verwendet. Herkömmlicherweise sind additive Fertigungsverfahren zum Herstellen großer Stückzahlen der jeweiligen Objekte kaum geeignet, da die Herstellung einzelner Objekte viel Zeit in Anspruch nimmt. Bei additiven Fertigungsverfahren, insbesondere beim 3-D-Drucken, wird das herzustellende Objekt aus einer Vielzahl sehr dünner übereinander angeordnete Schichten aufgebaut, die oftmals nur einige Mikrometer dick sind. Daher ist die Herstellung insbesondere großer Objekte zeitaufwendig und hat sich deswegen zur Herstellung großer Stückzahl noch nicht durchgesetzt.

Aus der DE 10 2018 116 790 A1 ist beispielsweise ein Verfahren bekannt, bei dem schichtweise ein Gegenstand aufgebaut und hergestellt wird. Dies kann beispielsweise durch ein Sinter-Verfahren oder ein Sinter-ähnliches Verfahren geschehen oder in dem selektiv beispielsweise mit Laser-Unterstützung einen Werkstoff durch Abschmelzen auf die jeweils vorherige Schicht aufzubringen. Während oder nach diesem Verfahren wird bei dem beschriebenen Stand der Technik das jeweils hergestellte Produkt vermessen.

Vom MIT wurde ein 3-dimensionales Druckverfahren entwickelt, das in der US 2018/281295 A1 veröffentlicht wurde und als "Rapid Liquid Printing" (RLP) bezeichnet wird. Ein ähnliches Verfahren ist der US 2016/0067918 A1 zu entnehmen. Dabei wird das herzustellende Objekt in einem Behälter erzeugt, der eine Gelsuspension oder ein anderes Material als Stützmaterial beinhaltet, das mit dem Fertigungsmaterial nicht chemisch reagiert. Es dient ausschließlich zum Stützen des Fertigungsmaterials, solange dieses noch nicht ausgehärtet oder ausreichend vernetzt ist. Bei dem Verfahren wird das Fertigungsmaterial in einem fließfähigen Zustand, beispielsweise flüssig oder gelförmig, an die gewünschten Positionen innerhalb des Stützmaterials eingebracht. Dazu wird die Einbringnadel verwendet, die in drei Richtungen verschoben werden kann, die linear unabhängig voneinander sind. Aufgrund der Dichteverhältnisse zwischen dem Fertigungsmaterial und dem Stützmaterial verbleibt das eingebrachte Fertigungsmaterial an der jeweiligen Position, sodass auf besonders schnelle Weise 3-dimensionale Objekte gedruckt werden können, indem das Fertigungsmaterial an die gewünschten Stellen gebracht wird und anschließend dort vernetzt, erstarrt oder aushärtet. Dieses Verfahren ist gegenüber bisherigen 3-D-Druckverfahren deutlich schneller und ermöglicht es auch flexible oder elastische Gegenstände herzustellen. Zudem erlaubt es, eine Vielzahl an Materialien zu verdrucken, die nicht speziell für additive Fertigungsverfahren optimiert wurden, sondern sich in konventionellen Gussprozessen etabliert haben. Dazu zählen beispielsweise biokompatible Silikone und insbesondere Elastomere mit niedrigen Shore-Härten zur Herstellung elastischer Gegenstände.

Im Vergleich zu herkömmlichen 3-D-Druckverfahren weisen RLP-Verfahren den Nachteil auf, dass es bei dem Fertigungsmaterial nach dem Einbringen in das Stützmaterial noch zu Verformungen kommt. Für das Fertigungsmaterial ändern sich beim Verlassen der Einbringnadel durch die wenigstens einer Einbringöffnung die Druckverhältnisse. Da das Fertigungsmaterial in einem fließfähigen Zustand eingebracht wird, kann es sich diesen veränderten Druckverhältnissen anpassen und wird sich verformen. Bei einigen Fertigungsmaterialien, insbesondere solchen, die in Form mehrerer Komponenten eingebracht werden, kommt es nach dem Einbringen in das Stützmaterial zu chemischen Reaktionen, beispielsweise Vernetzungen. Auch dadurch können sich Fließeigenschaften oder das Volumen des Materials verändern, was ebenfalls zu Verformungen des eingebrachten Fertigungsmaterials führt.

Die Schwierigkeit der RLP-Verfahren besteht daher unter anderem darin, diese Fließeigenschaften und die daraus resultierenden Verformungen des Fertigungsmaterials möglichst gut zu kennen und bei der Herstellung des 3-dimensionalen Objektes zu berücksichtigen. Dies ist besonders bei Fertigungsmaterialien eine Herausforderung, die nach dem Aushärten elastisch oder flexibel sind. In diesem Fall ist auch das hergestellte 3-dimensionale Objekt zumindest abschnittsweise flexibel oder elastisch, sodass sich die Kontur nach dem Entfernen des Objektes aus dem Stützmaterial verändert. Bei einem solchen 3-dimensionalen Objekt kann es sich beispielsweise um den Liner für eine Prothese handeln, der elastisch ausgebildet ist. Wird er oder ein anderes elastisches oder flexibles Objekt aus dem Stützmaterial entnommen, fällt er der Schwerkraft folgend in sich zusammen, sodass die hergestellte und gedruckte Kontur nicht mehr eindeutig und somit nicht messbar ist.

Der Erfindung liegt daher die Aufgabe zugrunde, ein gattungsgemäßes Verfahren zu verbessern und die Nachteile aus dem Stand der Technik zu beheben oder zumindest abzumildern.

Die Erfindung löst die gestellte Aufgabe durch ein Verfahren gemäß dem Oberbegriff des Anspruchs 1, das sich dadurch auszeichnet, dass die Kontur und/oder die Position wenigstens eines Teils des 3-dimensionalen Objektes innerhalb des Stützmaterials mittels wenigstens eines Sensors erfasst wird. Vorzugsweise wird die Kontur des ganzen 3-dimensionalen Objektes mittels des wenigstens einen Sensors erfasst.

Der wenigstens eine Sensor ist folglich in der Lage, die Kontur, also die geometrische Form, des 3-dimensionalen Objektes zumindest teilweise, vorzugsweise jedoch vollständig, zu erfassen. Dies bedeutet, dass der Sensor Sensordaten erfasst, aus denen sich Rückschlüsse auf die Kontur ziehen lassen. Es ist nicht notwendig, dass der Sensor direkt die Kontur erfasst. Die Sensordaten werden von dem wenigstens einen Sensor vorzugsweise an eine elektrische Steuerung, insbesondere eine elektronische Datenverarbeitungseinrichtung übermittelt. Diese ist in der Lage mittels eines durch die Steuerung ausgeführten Algorithmus aus den Sensordaten die Kontur zu ermitteln. Bei der so ermittelten Kontur handelt es sich um die Ist-Kontur, die dann beispielsweise in der elektrischen Steuerung mit einer Soll-Kontur, die vorzugsweise in einem elektronischen Datenspeicher hinterlegt ist, auf den die elektrische Steuerung zugreifen kann, verglichen. Auf diese Weise lässt sich das Ergebnis des Verfahrens und insbesondere die Qualität des 3-dimensionalen Objektes bereits im Stützmaterial erkennen, sodass Druckparameter, beispielsweise eine Austrittsrate des Fertigungsmaterials durch die Einbringöffnung der Einbringnadel, eine Geschwindigkeit, mit der die Einbringnadel durch das Stützmaterial bewegt wird, oder der Weg, entlang dessen die Einbringnadel bewegt wird, angepasst und verändert werden können. Auch kann so die genaue Position des 3-dimensionalen Objektes erfasst werden, sodass beispielsweise weitere Elemente angedruckt werden können. Die Position kann sich insbesondere bei längeren Druckvorgängen ändern, wenn das herzustellende Objekt im Gel aufgrund der Einwirkung der Schwerkraft leicht absackt.

Vorzugsweise befindet sich in dem Stützmaterial wenigstens ein Objekt, dessen Kontur und/oder Position innerhalb des Stützmaterials bekannt ist oder mittels des wenigstens einen Sensors erfasst wird. Ein solches Objekt befindet sich bevorzugt bereits in dem Stützmaterial, bevor mit dem Einbringen des Fertigungsmaterials in das Stützmaterial begonnen wird. Die Kontur und/oder die Position wird vorzugsweise mittels des wenigstens einen Sensors erfasst und gespeichert. Bei einem solchen Objekt handelt es sich beispielsweise um einen Gegenstand, der mit dem Fertigungsmaterial verbunden werden soll oder an den das Fertigungsmaterial angedruckt werden soll. Angedruckt ist das Fertigungsmaterial, wenn es im fließfähigen und/oder ausgehärteten Zustand mit dem Objekt in Kontakt ist. Es kann sich bei dem Objekt beispielsweise um eine Linerkappe handeln, die mit einem elastischen Linermaterial, das im fließfähigen Zustand des Fertigungsmaterials bildet, verbunden wird.

Alternativ kann das Objekt auch verwendet werden, um eine Außenfläche und/oder eine Innenfläche des herzustellenden 3-dimensionalen Objektes herzustellen. Wenn eine solche Oberfläche beispielsweise besonders glatt sein oder eine bestimmte Oberflächenstruktur aufweisen soll, kann das Objekt als "Form" verwendet werden. Das Fertigungsmaterial wird im fließfähigen Zustand an das Objekt angedruckt und nach dem Aushärten wieder von dem Objekt entfernt. Die Struktur der Fläche, mit der das Objekt mit dem Fertigungsmaterial in Kontakt kommt, wird auf die jeweilige Oberfläche des 3-dimensionalen Objektes übertragen.

Vorzugsweise beinhaltet die Kontur des 3-dimensionalen Objektes eine Außenfläche und/oder eine Innenflächen und/oder Wanddicke des Objektes. Besonders bevorzugt wird die Kontur des 3-dimensionalen Objektes durch die gesamte Oberfläche des Objektes gebildet. Dies beinhaltet besonders bevorzugt auch die Oberfläche von Hohlräumen, die das Objekt aufweist. Insbesondere orthopädietechnische Objekte, beispielsweise Prothesenliner, verfügen über eine Außenfläche und eine Innenfläche. Ein Prothesenliner wird zwischen einem Amputationsstumpf und einem Prothesenschaft getragen und dient beispielsweise zur Polsterung. Der Prothesenliner ist in der Regel aus einem elastischen Material hergestellt und wird bevorzugt individuell an den Amputationsstumpf und insbesondere dessen Kontur angepasst. Der Prothesenliner verfügt über eine Innenfläche, die im angelegten Zustand dem Amputationsstumpf des Trägers des Prothesensystems, dessen Teil der Prothesenliner ist, zugewandt ist. Die Innenfläche kommt mit der Haut des Trägers in Kontakt. Der Prothesenliner verfügt zudem über eine Außenfläche, die im angelegten Zustand dem Prothesenschaft zugewandt oder zumindest dem Amputationsstumpf abgewandt ist. Vorzugsweise beinhaltet die Kontur sowohl Außenfläche des Prothesenliners als auch die Innenfläche.

In einer bevorzugten Ausgestaltung weist der wenigstens eine Sensor wenigstens einen optischen Sensor, beispielsweise eine Kamera, auf. Die Kontur des 3-dimensionalen Objektes wird in diesem Fall optisch erfasst. Der optische Sensor kann vorzugsweise sichtbares Licht detektieren. Es ist jedoch auch möglich einen optischen Sensor zu verwenden, der elektromagnetische Strahlung außerhalb des optischen Spektralbereiches, beispielsweise UV-Strahlung oder Infrarotstrahlung, detektieren kann.

Vorzugsweise ist das Stützmaterial für elektromagnetische Strahlung, die von dem optischen Sensor detektiert werden kann, transparent, zumindest jedoch teiltransparent. Insbesondere für den Fall, dass das Fertigungsmaterial nach dem Aushärten für diese elektromagnetische Strahlung nicht transparent ist, bietet sich die Verwendung eines optischen Sensors an. In einer bevorzugten Ausgestaltung wird das 3-dimensionale Objekt zumindest in dem Bereich, dessen Kontur erfasst werden soll, mit einer elektromagnetischen Strahlung beleuchtet, die vorzugsweise der vom optischen Sensor detektierbaren elektromagnetischen Strahlung entspricht. Dabei können besondere Beleuchtungsmuster, beispielsweise parallele Beleuchtungsstreifen, die vorzugsweise eine bekannte Breite und einen bekannten Abstand voneinander aufweisen, verwendet werden. Der optische Sensor detektiert dann die vom 3-dimensionalen Objekt reflektierten Anteile dieser Beleuchtung und die elektrische Steuerung kann aus dem detektierten Muster Rückschlüsse auf die Kontur des 3-dimensionalen Objektes ziehen. Ist das Objekt für die elektromagnetische Strahlung zumindest teiltransparent ausgebildet, können der Sender und der Empfänger auch auf gegenüberliegenden Seiten des Objektes angeordnet werden. Dies bedeutet, dass die elektromagnetische Strahlung auf dem Weg vom Sender zum Empfänger wenigstens einmal durch das Fertigungsmaterial geleitet wird. So können auch Informationen über das Objekt aus der Absorption der elektromagnetischen Strahlung erhalten werden. So sind beispielsweise Verfahren anwendbar, die aus der Tomographie bekannt sind.

Alternativ oder zusätzlich dazu weist der wenigstens eine Sensor wenigstens einen Ultraschallsensor auf. In einer besonders bevorzugten Ausgestaltung handelt es sich bei dem Stützmaterial um ein Ultraschallgel, das auch verwendet werden kann, um als Schnittstelle zwischen dem Ultraschallkopf und dem Körper eines Trägers bei einer Ultraschalluntersuchung zu dienen. Wenn der wenigstens eine Sensor wenigstens einen Ultraschallsensor aufweist, der zum Detektieren von Ultraschallwellen eingerichtet ist, ist es von Vorteil, wenn der Sensor selbst oder zumindest die Vorrichtung, die zur Durchführung eines derartigen Verfahrens verwendet wird, auch eine Ultraschallquelle aufweist. In der Regel werden Ultraschallsender und Ultraschallempfänger in einem gemeinsamen Gehäuse angeordnet und gemeinsam als Ultraschallkopf bezeichnet. Der Ultraschallsender und der Ultraschallempfänger können derselbe Ultraschallwandler sein.

Ein Vorteil bei der Verwendung wenigstens eines Ultraschallsensors besteht darin, dass die Ultraschallwellen nicht nur an der Außenfläche des Objektes innerhalb des Stützmaterials reflektiert werden, sondern auch in das ausgehärtete oder aushärtende Fertigungsmaterial eindringen und auch an der nächsten Grenzfläche zwischen zwei unterschiedlichen Materialien, also beispielsweise der Innenfläche des 3-dimensionalen Objektes reflektiert werden. Die vom Ultraschallsensor empfangenen und an den Grenzflächen zwischen den unterschiedlichen Materialien reflektierten Ultraschallwellen enthalten folglich Informationen sowohl über die Außenfläche als auch über die Innenfläche des 3-dimensionalen Objektes, sodass auf diese Weise die gesamte Kontur des 3-dimensionalen Objektes erfassbar ist.

Vorzugsweise wird in der wenigstens eine Sensor relativ zu dem 3-dimensionalen Objekt bewegt, um die Kontur zu erfassen. Dies ist unabhängig von der Art des Sensors und insbesondere für optische Sensoren und für Ultraschallsensoren von Vorteil. Der wenigstens eine Sensor kann ein mobiler Handsensor, beispielsweise ein optischer Scanner oder ein bewegbare Ultraschallsensor, beispielsweise ein Ultraschallkopf sein. Alternativ oder zusätzlich dazu ist wenigstens einen Sensor entlang einer vorbestimmten Bahn bewegbar. Diese Bahn kann beispielsweise in Form einer Schiene oder eines Schienensystems vorhanden sein und zumindest teilweise, vorzugsweise jedoch vollständig um den Behälter herumführen, in dem sich das Stützmaterial befindet und in dem das 3-dimensionale Objekt gedruckt wird. Alternativ dazu kann der Sensor auch an einem bewegbaren Arm befestigt sein, der beispielsweise ein Roboterarm ist und durch einen Motor angetrieben und beispielsweise durch eine elektrische Steuerung gesteuert entlang einer vorbestimmten Bahn bewegt werden kann.

Vorteilhafterweise ist wenigstens ein Sensor in dem Stützmaterial positioniert. Der Sensor ist vorzugsweise an einer Halterung im Innern des Behälters, in dem sich das Stützmaterial befindet, befestigt. Er verfügt vorzugsweise über eine elektrischen Energiespeicher, beispielsweise eine Batterie. Der Sensor verfügt vorteilhafterweise über eine drahtlose Kommunikationsschnittstelle, beispielsweise einen WLAN-Anschluss oder eine Bluetooth-Schnittstelle, mit der der Sensor in der Lage ist, drahtlos mit einer elektrischen Steuerung, insbesondere einer elektronischen Datenverarbeitungseinrichtung zu kommunizieren und Messwerte, die der Sensor aufnimmt, an diese elektrische Steuerung weiterzuleiten. Alternativ oder zusätzlich dazu können Energieversorgungskabel, beispielsweise Stromkabel und/oder Datenübertragungskabel durch die Wand des Behälters, in dem sich das Stützmaterial befindet, durchgeführt werden, um den innerhalb des Behälters angeordneten Sensor mit der benötigten Energie zu versorgen und/oder eine Kommunikation zu ermöglichen. Vorzugsweise wird der innerhalb des Stützmaterials positionierte Sensor durch das Fertigungsmaterial teilweise umgeben, sodass es ihm besonders einfach möglich ist, eine Innenfläche des 3-dimensionalen Objektes zu erfassen.

Bevorzugt wird der wenigstens eine Sensor derart in dem Stützmaterial positioniert, dass er zumindest teilweise umdruckt wird und so die Innenfläche des 3-dimensionalen Objektes erfassen kann. Vorzugsweise ist der wenigstens eine Sensor bereits im Stützmaterial positioniert, bevor das Einbringen des Fertigungsmaterials in das Stützmaterial beginnt. Ein außerhalb des Behälters, in dem sich das Stützmaterial befindet, angeordneter Sensor muss immer auch die Behälterwand und somit mehr Grenzflächen durchdringen, an denen beispielsweise Messstrahlung, beispielsweise elektromagnetische Strahlung oder Ultraschall, reflektiert oder gebrochen werden kann. Ein innerhalb des Stützmaterials positionierter Sensor hat daher den Vorteil, dass weniger Grenzflächen vorhanden sind.

Vorzugsweise wird die Kontur nach oder während dem Aushärten des Fertigungsmaterials erfasst.

Besonders bevorzugt ist an der Einbringnadel wenigstens ein zusätzlicher Sensor, vorzugsweise ein optischer Sensor, besonders bevorzugt eine Kamera, angeordnet, durch den das durch die Einbringnadel eingebrachte Fertigungsmaterial erfasst wird. Auf diese Weise wird nicht nur die Kontur des 3-dimensionalen Gegenstandes erfasst. Vielmehr ist es auch möglich, den Austritt des Fertigungsmaterials aus der Einbringöffnung der Einbringnadel zu überwachen. So können beispielsweise Informationen über das Fließverhalten des Fertigungsmaterials gesammelt werden. Diese können bei der Ermittlung von Druckparametern für weitere 3-dimensionale Objekte herangezogen werden. Zudem kann überwacht werden, ob das Fertigungsmaterial ordnungsgemäß in das Stützmaterial eingebracht wird. Dies betrifft beispielsweise einen blasenfreien Materialstrom.

Vorzugsweise erfolgt das Erfassen der Kontur und/oder der Position des 3-dimensionalen Objektes bereits während des Druckprozesses, also während des Einbringens des Fertigungsmaterials in das Stützmaterial. Auf diese Weise können die erfassten Daten, die Informationen über Kontur und/oder Position der bereits gedruckten Teile und/oder Austrittseigenschaften des Fertigungsmaterials aus der Einbringnadel beinhalten, der elektrischen Steuerung übermittelt werden. Sie können so direkten Einfluss auf das Druckverfahren nehmen und gegebenenfalls auftretende Fehler können schnell und unkompliziert korrigiert werden. Beispielsweise könnte der Druck bei Problemen vorzeitig abgebrochen werden oder aber die bestimmte Parameter, wie etwa die Wandstärke, könnte durch eine Änderung der Druckparameter, wie etwa die Geschwindigkeit der Einbringnadel, nachgeregelt werden. So eine Nachregelung kann insbesondere erforderlich sein, wenn Eigenschaften des Stützmaterials und/oder des Fertigungsmaterials von erwarteten Eigenschaften abweichen oder andere Einflussfaktoren das Druckergebnis verändern.

Eine konstante Überwachung des Druckprozesses insbesondere in Hinblick auf die Position des herzustellenden 3-dimensionalen Objektes im Stützmaterial kann auch die Genauigkeit erhöhen, mit denen weitere Objekte an das 3-dimensionale Objekt angedruckt werden. Dies ist insbesondere wichtig, da die Objekte über die Dauer des Druckprozesses im Stützmaterial absinken können. Soll beispielsweise eine Dichtlippe an einen Liner angedruckt werden, so ist es wichtig dies genau in der richtigen Höhe vorzunehmen. Auch die Erfassung weiterer Objekte, die vorzugsweise vorab in das Stützmaterial eingebracht wurden, kann einen Vorteil bieten, etwa um das Andrucken an diese zu ermöglichen

Die Erfindung löst die gestellte Aufgabe zudem durch eine Vorrichtung zum Durchführen eines hier beschriebenen Verfahrens, die einen Behälter, der mit dem Stützmaterial gefüllt oder füllbar ist, und wenigstens eine Einbringnadel aufweist, durch die das Fertigungsmaterial in den Behälter einbringbar ist. Die Vorrichtung verfügt über wenigstens einen Sensor, mittels dessen die Kontur wenigstens eines Teils des 3-dimensionalen Objektes erfassbar ist, das sich in dem Behälter befindet. Vorzugsweise verfügt die Vorrichtung über eine elektrische Steuerung, besonders bevorzugt eine elektronische Datenverarbeitungseinrichtung, die zur Verarbeitung der Sensordaten, die von dem wenigstens einen Sensor erfasst werden, eingerichtet ist. Der Sensor verfügt über eine Kommunikationsschnittstelle, die mit einer entsprechenden Schnittstelle der elektrischen Steuerung zusammenwirkt, sodass der Sensor seine Daten an die elektrische Steuerung übermittelt. Die Sensordaten enthalten Informationen über die Kontur wenigstens eines Teils, vorteilhafterweise jedoch des ganzen 3-dimensionalen Objektes. Diese Kontur wird vorzugsweise in der elektrischen Steuerung aus den Daten extrahiert und für die weitere Verarbeitung aufbereitet. Die Vorrichtung verfügt in einer bevorzugten Ausgestaltung über einen elektronischen Datenspeicher, in dem Daten, beispielsweise Soll-Daten für die Kontur des Objektes, in elektronischer Form hinterlegt sind. Die elektrische Steuerung kann auf den elektronischen Datenspeicher zugreifen und die Soll-Daten abfragen und beispielsweise mit aus den Sensordaten extrahierten Daten der Kontur des Objektes, der sogenannten Ist-Kontur, abgleichen.

Mit Hilfe der beigefügten Zeichnungen werden nachfolgend einige Ausführungsbeispiele der vorliegenden Erfindung näher erläutert. Es zeigen:

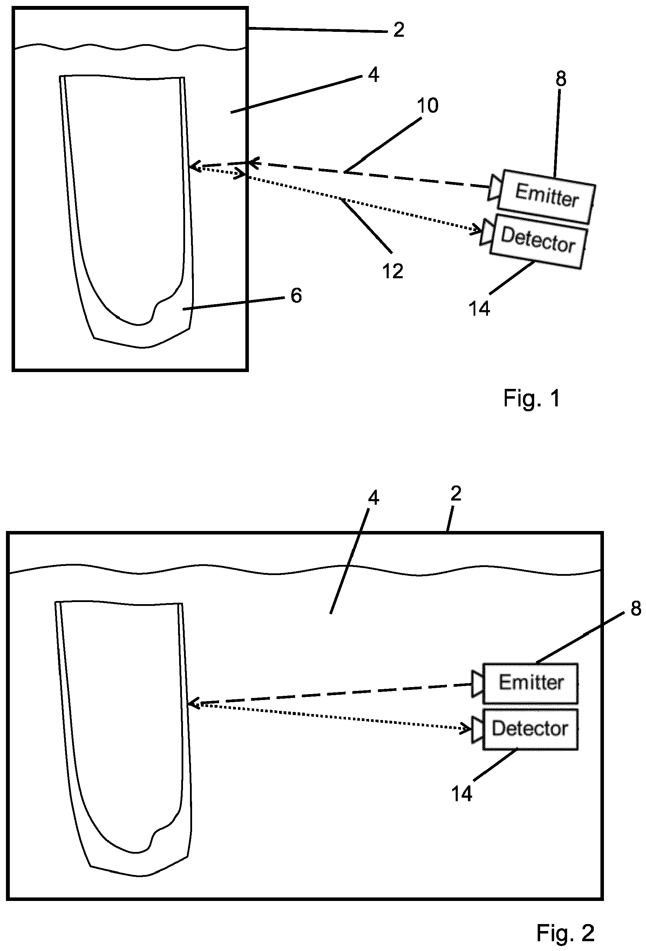

Figuren 1 bis 9 - schematische Darstellungen unterschiedlicher Vorrichtungen zum Durchführen von Verfahren gemäß Ausführungsbeispielen der vorliegenden Erfindung.
Figur 1 zeigt einen Behälter 2, in dem sich Stützmaterial 4 befindet. In dem Stützmaterial 4 ist ein 3-dimensionales Objekt 6 dargestellt, das vermessen werden soll und in dem Behälter 2 hergestellt wurde. Dazu ist ein Emitter 8 dargestellt, der eine Messstrahlung 10 aussendet. Die Messstrahlung 10 wird an der Wand des Behälters 2 gebrochen und trifft in dem Stützmaterial 4 auf das Objekt 6. Dort wird sie reflektiert und tritt als reflektierte Strahlung 12 aus dem Behälter aus. Sie wird von einem Detektor 14 detektiert. Der Emitter 8 und der Detektor 14 sind außerhalb des Behälters 2 angeordnet. Sie sind im gezeigten Ausführungsbeispiel nicht beweglich ausgebildet. Emitter 8 und Detektor 14 bilden gemeinsam den Detektor, der die Kontur und/oder die Position des Objektes 6 innerhalb des Behälters 2 erfasst. Die Messstrahlung 10 kann beispielsweise eine Streifenbeleuchtung sein.
Figur 2 zeigt eine ähnliche Ausführungsform. Der Emitter 8 und der Detektor 14 sind innerhalb des Behälters 2 und innerhalb des Stützmaterials 4 positioniert. Dies hat den Vorteil, dass weder die Messstrahlung 10 noch die reflektierte Strahlung 12 an der Wand des Behälters 2 gebrochen werden.

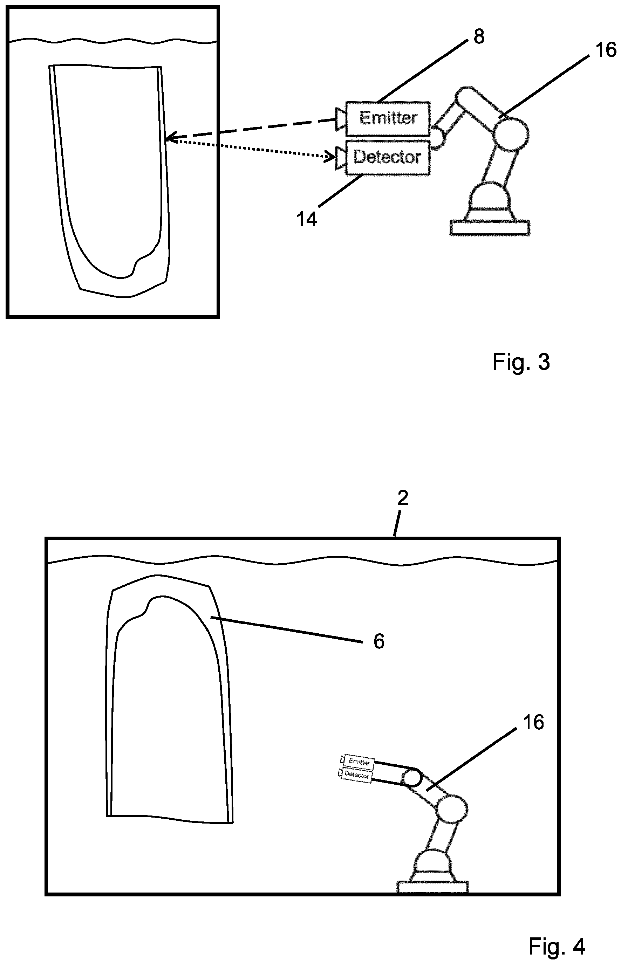

Figur 3 zeigt eine Ausführungsform ähnlich der Figur 1. Auch hier sind der Emitter 8 und der Detektor 14 außerhalb des Behälters 2 angeordnet. Beide gemeinsam sind an einem Aktuator 16 angeordnet, der im gezeigten Ausführungsbeispiel als Roboterarm ausgebildet ist. Dadurch werden Emitter 8 und Detektor 14 gemeinsam bewegbar und es wird möglich, das Objekt 6 zu scannen und von verschiedenen Seiten und aus unterschiedlichen Perspektiven zu erfassen. Die Messstrahlung 10 und die reflektierte Strahlung 12 sind der Einfachheit halber nicht als an der Wand des Behälters gebrochen dargestellt. Dennoch wird die Strahlung bei jedem Durchtritt durch die Grenzflächen gebrochen.
Figur 4 zeigt den Aktuator 16 mit Emitter 8 und Detektor 14 innerhalb des Behälters 2. Das Objekt 6 ist wie in den anderen Figuren als Liner dargestellt. Anders als in den Figuren 1 bis 3 ist der Liner in Figur 4 mit offenen Ende 18, das das proximale Ende ist, nach unten hergestellt worden. Dadurch wird es einfacher, das 3-dimensionale Objekt 6 aus dem Behälter 2 zu entfernen.

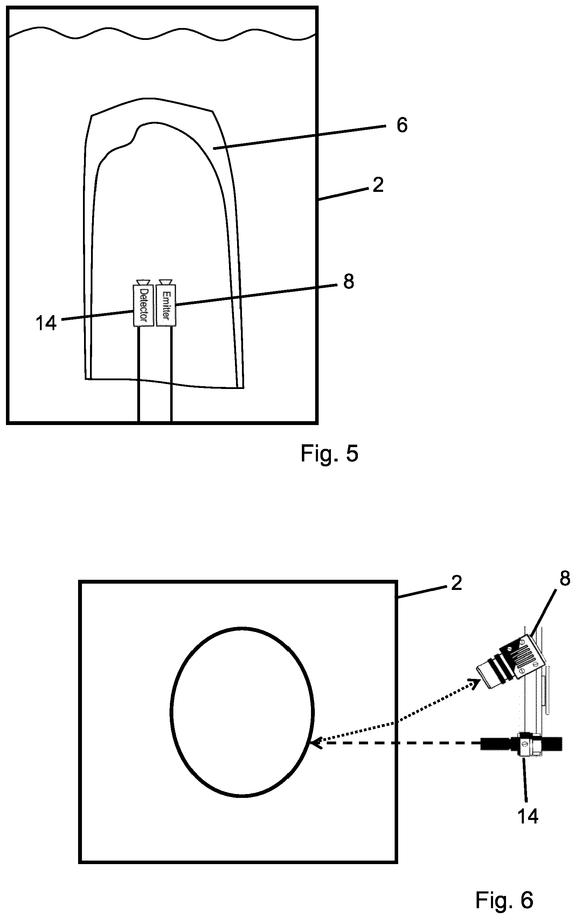

In Figur 5 sind der Emitter 8 und der Detektor 14 innerhalb des Objektes 6 angeordnet. Statt wie in den anderen Figuren die Außenseite zu erfassen, wird durch die Ausgestaltung in Figur 5 die Innenseite des Objektes 6 erfasst.

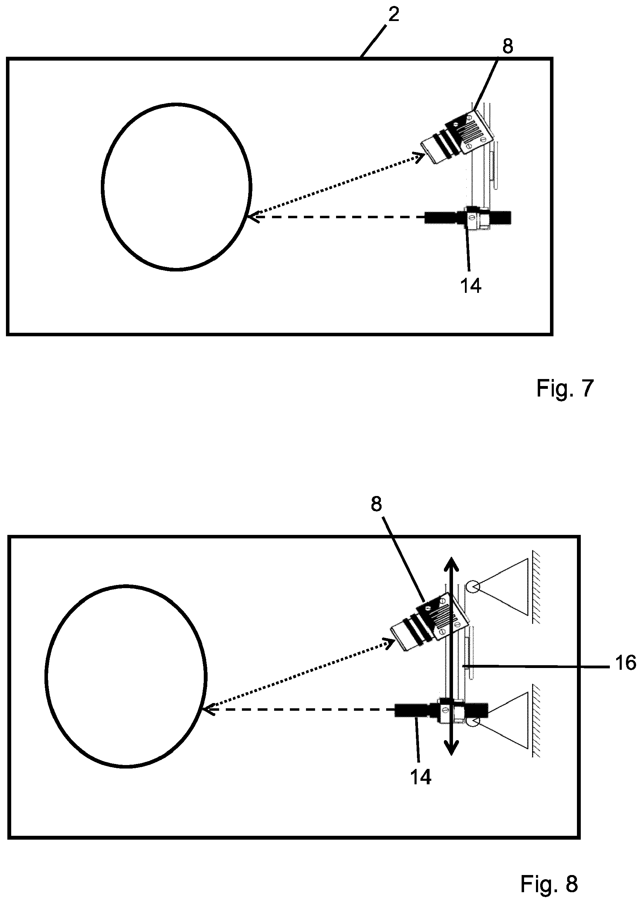

Figur 6 entspricht der Darstellung aus Figur 1 mit einem anderen Emitter 8 und einem anderen Detektor 14. Der Emitter 8 ist in diesem Falle in Laser, wobei die Bestimmung der Kontur und/oder der Position des Objektes im gezeigten Ausführungsbeispiel mittels Triangulation erfolgt. Beide sind außerhalb des Behälters 2 angeordnet. Figur 7 zeigt den Emitter 8 und den Detektor 14 innerhalb des Behälters 2. In Figur 8 sind Emitter 8 und Detektor 14 an einem gemeinsamen Aktuator 16 angeordnet, über den sie gemeinsam bewegbar sind.

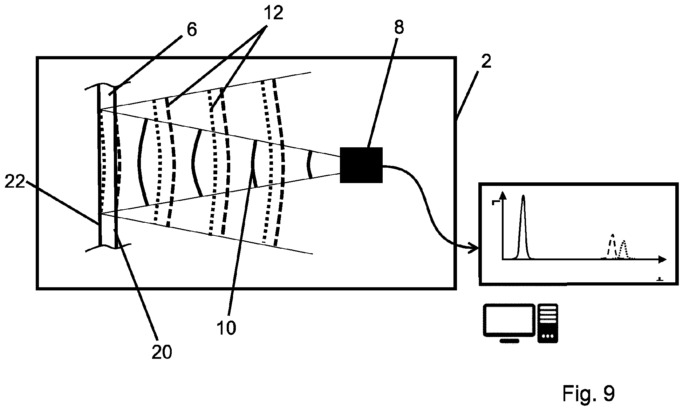

Figur 9 zeigt eine andere Ausgestaltung. Innerhalb des Behälters 2 ist der Emitter 8, der als Ultraschallsender ausgebildet ist. Die Messstrahlung 10 wird in Form von Ultraschallwellen ausgesendet. Sie wird durch durchgezogene Linien dargestellt. Die Messstrahlung 10 trifft auf das Objekt 6 und wird von der Außenfläche 20 und der Innenfläche 22 reflektiert. Es entstehen zwei unterschiedliche reflektierte Strahlungen 12, die als gestrichelte und gepunktete Linie dargestellt sind. Der Emitter 8 ist gleichzeitig der Detektor 12 und sendet die Messdaten an eine elektrische Steuerung 24. In den Messdaten ist der Sendepeak 26 und die beiden Reflektionspeaks 28 zu erkennen.

### Bezugszeichenliste

2 Behälter
4 Stützmaterial
6 Objekt
8 Emitter
10 Messstrahlung
12 Reflektierte Strahlung
14 Detektor
16 Aktuator
18 Offenes Ende
20 Außenfläche
22 Innenfläche
24 Elektrische Steuerung
26 Sendepeak
28 Reflektionspeak

## Patentansprüche

1. Verfahren zum Herstellen eines 3-dimensionalen Objektes mittels eines additiven Fertigungsverfahrens, bei dem aus wenigstens einer Einbringöffnung einer Einbringnadel wenigstens ein Fertigungsmaterial in einem fließfähigen Zustand in ein Stützmaterial eingebracht wird und danach aushärtet, wobei das Fertigungsmaterial nach dem Aushärten elastisch ist,
**dadurch gekennzeichnet, dass**
die Kontur und/oder die Position wenigstens eines Teils des 3-dimensionalen Objektes innerhalb des Stützmaterials mittels wenigstens eines Sensors erfasst wird.

2. Verfahren nach Anspruch 1, **dadurch gekennzeichnet, dass** die Kontur des ganzen 3-dimensionalen Objektes mittels des wenigstens einen Sensors erfasst wird.

3. Verfahren nach Anspruch 1 oder 2, **dadurch gekennzeichnet, dass** sich in dem Stützmaterial wenigstens ein Objekt befindet, dessen Kontur und/oder Position innerhalb des Stützmaterial bekannt ist oder mittels des wenigstens einen Sensors erfasst wird.

4. Verfahren nach Anspruch 1 oder 2, **dadurch gekennzeichnet, dass** die Kontur des 3-dimensionalen Objektes eine Außenfläche und/oder eine Innenfläche und/oder Wanddicke des Objektes beinhaltet.

5. Verfahren nach einem der vorstehenden Ansprüche **dadurch gekennzeichnet, dass** der wenigstens eine Sensor wenigstens einen optischen Sensor, beispielsweise eine Kamera, aufweist.

6. Verfahren nach einem der vorstehenden Ansprüche, **dadurch gekennzeichnet, dass** der wenigstens eine Sensor wenigstens einen Ultraschallsensor aufweist.

7. Verfahren nach einem der vorstehenden Ansprüche, **dadurch gekennzeichnet, dass** wenigstens ein Sensor relativ zu dem 3-dimensionalen Objekt bewegt wird, um die Kontur zu erfassen.

8. Verfahren nach einem der vorstehenden Ansprüche, **dadurch gekennzeichnet, dass** wenigstens ein Sensor in dem Stützmaterial positioniert ist.

9. Verfahren nach Anspruch 8, **dadurch gekennzeichnet, dass** wenigstens ein Sensor derart in dem Stützmaterial positioniert ist, dass er zumindest teilweise umdruckt wird und so die Innenfläche des 3-dimensionalen Objektes erfassen kann.

10. Verfahren nach einem der vorstehenden Ansprüche, **dadurch gekennzeichnet, dass** die Kontur nach oder während dem Aushärten des Fertigungsmaterials erfasst wird.

11. Verfahren nach einem der vorstehenden Ansprüche, **dadurch gekennzeichnet, dass** an der Einbringnadel wenigstens ein zusätzlicher Sensor, vorzugsweise ein optischer Sensor, besonders bevorzugt eine Kamera, angeordnet ist, durch den das durch die Einbringnadel eingebrachte Fertigungsmaterial erfasst wird.

12. Verfahren nach einem der vorstehenden Ansprüche, **dadurch gekennzeichnet, dass** das Erfassen der Kontur und/oder der Position während des Einbringens des Fertigungsmaterials in das Stützmaterial erfolgt und vorzugsweise Einfluss auf das weitere Einbringen hat.

13. Vorrichtung zum Durchführen eines Verfahrens gemäß einem der vorstehenden Ansprüche, wobei die Vorrichtung einen Behälter, der mit Stützmaterial gefüllt oder füllbar ist, und wenigstens eine Einbringnadel, durch die ein Fertigungsmaterial in den Behälter einbringbar ist, aufweist **dadurch gekennzeichnet, dass** die Vorrichtung wenigstens einen Sensor aufweist, mittels dessen die Kontur wenigstens eines Teils eines 3-dimensionalen Objektes erfassbar ist, das sich in dem Behälter befindet.
